# EUROPEAN PATENT APPLICATION

(11) **EP 1 640 345 A1**
(43) Date of publication of application: **29.03.2006**
(21) Application number: 03816969.4
(22) Date of filing: 07.08.2003
(51) Int. Cl.: C02F 1/68

(54) **METHOD OF PRODUCING VIBRATION INFORMATION FOR SUPPORTING VITAL FORCE OF ORGANISM**

(30) Priority: 16.05.2003 JP 2003175585
(71) Applicant: Lidic Inc., Osaka-shi, Osaka 534-0025 (JP)
(72) Inventor: ODA, Shinichi, Lidic Inc., Osaka-shi, Osaka 534-0025 (JP)
(74) Representative: Dunleavy, Kevin James
(86) International application number: PCT/JP2003/010123
(87) International publication number: WO 2004/101445

(57) **Abstract**

The present invention aims at realizing mass production of an oscillation data recorded in oscillation data recording water for supporting vitality of living bodies on the industrial basis. Using an oscillation data in-printer, the negative code, the positive code, and the intermediate code are respectively recorded in the oscillation data recording water, which are then mixed with each other by an equivalent volume. After further preparing the mixed water by a predetermined unit number, the prepared water units are further mixed together before completing the production process.

## Description

### FIELD OF THE INVENTION

The present invention relates to a method of generating oscillation data for supporting vitality of living bodies as the main object of the invention by enabling oscillation data to be recorded and preserved in the novel oscillation data recording water via an oscillation data recording device.

### BACKGROUND OF THE INVENTION

From early times, there has been an idea of enabling water to solely memorize specific data designating own properties of materials so as to make use of the stored data for medical treatments.

As an outstanding example, the "Homeopathy" medical treatment originated by Samuel Hahnnemann, a German physician, in the early 19th century, is well known. In recent years, there has been an increasing number of physicians practically applying the homeopathy medical treatment in particular.

The basic conceptions of the homeopathy medical treatment are as described below. For example, after repeatedly diluting solution extracted from a certain medical herb with water, molecules of the medical herb will soon disappear as viewed statistically. On the other hand, only data designating own physical properties of the medical herb remains in the water.

After administering the water-diluted solution to a patient, specific effect analogous to that of the medical herb will soon be generated in the patient's body.

The above-cited water properties have been evidenced by a huge number of clinical results from the homeopathy medical treatment gathered during the past two centuries.

There are a variety of theoretical explanations on the substance of clinical data. However, from a common viewpoint, generally, the majority of scientists are in favor of the opinion that the actual effect of the homeopathy medical treatment corresponds to "oscillation generated at certain electronic levels" present in materials or animate objects. Note that such data will be referred to as "oscillation data" hereinafter.

An art based on the idea for controlling the oscillation data by applying an electronic device has been present from early times. For a typical example, there is a diagnostic treatment device called an EAV (Electronic Acupuncture according to Dr. Voll), which has been spread in Germany and also in peripheral countries since the 1950's.

A wide variety of the above EAV devices have been developed in Germany to date, which have practically been operated in the existing medical treatment facilities. On the other hand, in the U.S.A., the Magnetic Resonance Analyzer (MRA) as another oscillation data measuring device derived from the technical background analogous to that of the above-cited EAVs was developed by Ronald J. Weinstock in 1989. The MRA technology can be confirmed by referring to the Japanese Patent Publication No. HEISEI-6-130008 (1994).

Further, as another art for enabling water to internally record oscillation data, a "living body's data transferring device" is disclosed in the Japanese Utility Model Publication No. 3010129.

Essentially, the above-cited method for implementing the homeopathy medical treatment eliminates the causes of the sickened symptoms by providing the patient's living body with oscillation data of materials, such as herb, that can generate a phenomenon analogous to the actual symptom borne by the living body after consumption thereof. In other words, it constitutes an approaching method for dealing with individual symptoms.

The above EAV medical treatment devices have made it possible to implement an "electronic homeopathy" medical treatment by means of providing living bodies with a homeopathy remedy (with oscillation data for a medical treatment) via low frequencies. Mostly, the EAV devices have already been operated by medical facilities in European countries.

If a patient suffers from a lever disease, for example, the MRA medical treatment device implements a means for solving the lever malfunction by providing the patient's living body with oscillation data designating a normal lever condition via low frequencies. As in the above homeopathy method, the MRA medical treatment method also deals with individual symptoms.

On the other hand, in recent years, so-called "alternative medical treatment (complementary medical treatment)" has drawn attentions of the concerned and spread out in the medical circles. From the holistic viewpoint, some of medical approaches aim at recovery of living bodies via natural curing effect by reinforcing physical strength of individual living bodies as a result of drawing attentions to the vitality intrinsic to individual living bodies.

As an extreme example, Adriane Fugh-Berman, MD., proposed a method to adopt continual exercises, such as stretching and jogging, as part of medical treatment in his book titled "Alternative Medicine" published in 1997.

Primarily, the term "vitality" can be defined as the source of power generated inside a living body so as to enable the living body to sustain his own life.

In terms of the study on vitality, although there are many approaches to define "how to enhance vitality", it appears that no decisive conclusion has ever been reached among a variety of theories for defining genuine properties of vitality itself

As a result of experiments executed by the inventors of the present invention, they found the actual existence of oscillation data generated by a living body solely in the alive condition, in which the living body continuously held on its living activities. The inventors further confirmed that the oscillation data disappeared simultaneously with termination of the living activities (death of the living body).

Theoretically, the living phenomenon can be defined as cyclically repeated living activities comprising oral consumption of foreign matters (foods and beverages) via external sources, conversion of the required energy source into his own energy, and excretion of excrements.

Insofar as the above cyclic processes for self-sustaining individual living bodies normally function, each living body can properly convert the required energy source into his own energy. Nevertheless, if the self-sustaining function ever weakens, the foreign matters remaining in the weakened living body turns into a heavy burden or it may cause the living body to incur unwanted disease.

As cited by a proverb saying that "medicine and food constitute an identical source for preserving a healthy life", the act of eating contributes to the sustenance and enhancement of individual vitality via consumption of foreign matters inside the living bodies and conversion of the required energy source into their own energy via the self-sustaining processes cited above.

As described above, subsequent to the above experiments, the inventors of the present invention confirmed that the above "oscillation data generated by a living body solely in the state in which the living body continuously sustains his living activities" was provided with his own intrinsic properties.

The fact that "any living body is provided with an intrinsic constitution and personality" has been clarified by analytical reports on the rejection of the organ planting and on the DNA analysis. Nevertheless, the inventors of the present invention eventually found that the oscillation data incorporating the "self" personality further incorporates such oscillation data intrinsic to the "self" lives in common with every living body.

The inventors of the present invention further confirmed that the personalities of all the living bodies could be categorized into 60 patterns.

The inventors of the present invention further confirmed via the experiments that the "self-sustaining" function of individual living bodies could be strengthened directly by consuming the previously invented oscillation data recording water (as per the Japanese Patent Application No. 20022-369039) containing the recorded "oscillation data generated by a living body solely in the state in which the living body continuously sustains his own living activities".

The above-referred function for "strengthening self-sustaining function" of individual living bodies concurrently realizes "direct strengthening of vitality" as well.

It should be noted that not only the verification to identify the actual compatibility between a certain food and a living body and also identify the perfect compatibility between the newly generated oscillation data and a living body could be implemented via the measurement by the MRA, but all the experiments pursued by the inventors of the present invention have also been implemented via the measurement by the MRA.

It was thus far considered impracticable to execute the MRA measuring process with a strict measuring level because time-aged variation normally occurred in the actual condition of the specimen.

Nevertheless, as a result of effective use of the above oscillation data recording water, it was possible to realize the recording of the oscillation data of the specimen at a fixed point. Hence, it has become practicable to implement an extremely precise analysis of the actual condition of a living body, and thus, it has become further practicable to very strictly analyze and grasp the influential degree against the vitality of individual living bodies by applying the MRA measuring system, thereby solving the pending issue.

By referring to the analytical result achieved by the inventors of the present invention via the MRA measuring system, it was eventually confirmed that the oscillation data generated by living bodies solely in the state of sustaining living activities could further enhance the influential effect against living bodies by applying three kinds of the inventive means as described hereinafter.

Hence, the primary object of the present invention is to artificially generate such an oscillation data incorporating a function capable of enhancing the vitality intrinsically held in individual living bodies by focusing attention to the basic properties of the oscillation data.

### SUMMARY OF THE INVENTION

The present invention has been achieved to solve the above problem, wherein the invention provides a method for generating an oscillation data mainly aiming at supporting the vitality of individual living bodies, wherein the oscillation data is recorded and preserved in the oscillation data recording water by applying an oscillation data recording device.

The inventive method for producing the oscillation data recording water comprises the following serial steps of;
producing three kinds of oscillation data recording water 3a, 3b, and 3c by recording them in a predetermined volume of oscillation data recording water 3 by means of an oscillation data in-printer 2 pertaining to an oscillation data measuring device 1 previously storing an oscillation data containing negative properties, another oscillation data containing positive properties, and the other oscillation data containing intermediate properties therein;
further producing the first mixed oscillation data recording water 4 by mixing said three kinds of oscillation data recording water 3a, 3b, and 3c with each other;
further producing predetermined plural units of the first mixed oscillation data recording water 3d by causing oscillation data of said first mixed oscillation data recording water 4 to be recorded in said oscillation data recording water 3 comprising a predetermined volume and predetermined plural units;
further producing the second mixed oscillation data recording water 6 by fully mixing predetermined plural units of said first mixed oscillation data recording water 3d with each other; and
completing production of said oscillation data recording water by cyclically repeating said reproducing, mixing, and reproducing processes by predetermined rounds, thereby enabling the oscillation data for supporting vitality of a living body to be recorded and preserved in the inventive oscillation data recording water.

Hence, according to the inventive method for generating oscillation data for supporting vitality of individual living bodies, by way of, for example, orally consuming the oscillation data recorded in the oscillation data recording water generates an advantage capable of enhancing vitality of individual living bodies without causing them to incur unwanted burden as a foreign matter.

### BRIEF DESCRIPTION OF THE DRAWINGS

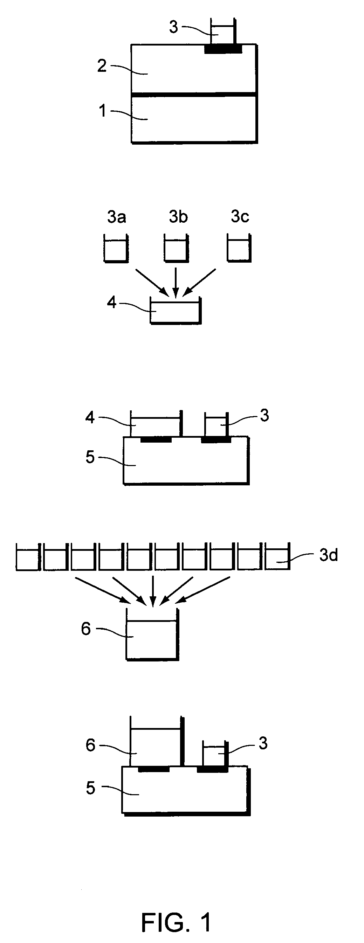

In the accompanying drawing; FIG 1 is a schematic block diagram showing production procedures when recording oscillation data in the oscillation data recording water by applying the method of the present invention for generating the oscillation data for supporting vitality of living bodies.

### DETAILED DESCRIPTION OF THE PREFERRED EMBODIMENTS

A practical form for embodying the present invention is described below by referring to the accompanying drawing illustrating the processes for actually producing the novel oscillation data recording water for recording oscillation data capable of supporting vitality of individual living bodies based on the inventive method for generating the above-cited oscillation data.

The reference numeral 1 shown in FIG 1 designates an oscillation data measuring device. The oscillation data measuring device 1 stores the previously encoded oscillation data. The oscillation data measuring device 1 generates optionally encoded oscillation data, which is concurrently capable of recording the encoded oscillation data in a predetermined volume of oscillation data recording water 3 by applying an oscillation data in-printer 2 attached to the oscillation data measuring device 1.

The above-cited optionally encoded oscillation data previously stored in the oscillation data measuring device 1 comprises the negative code, the positive code, and the intermediate code, which will be described afterwards.

More particularly, according to the oriental medicine, when categorizing the character of sample foods, generally, foods are categorized into two parts including the one prone to cool off the living body and the other one prone to warm the living body.

Hence, in the embodiment of the present invention, such a sample previously identified to be prone to cool off the living body via the oscillation data measuring device 1 has been encoded into the negative code, whereas such a sample previously identified to be prone to warm the living body has been encoded into the positive code. Such a sample identified to be intermediate between the above two samples has been encoded into the intermediate code.

As a result of the experiments pursued by the inventors of the present invention via the oscillation data measuring device 1, it was confirmed that oscillation data generated from a living body continuously sustaining living activities concurrently contained the cathode character and the anode character with an equally balanced proportion.

Production procedures are described below. First, using an oscillation data in-printer 2 attached to the oscillation data measuring device 1, the negative code designating the oscillation data bearing the negative property, the positive code designating the oscillation data bearing the positive property, and the intermediate code designating the oscillation data bearing the intermediate property, which are previously being stored in the above oscillation data measuring device 1, are respectively recorded in the oscillation data recording water 3 previously prepared in a glass beaker 7 by a predetermined volume (10ml for example) before eventually producing three kinds of the oscillation data recorded water comprising 3a, 3b, and 3c.

Next, by mixing the above-cited three kinds of the oscillation data recorded water 3a, 3b, and 3c, with each other in another glass beaker 7a, the first mixed oscillation data recorded water 4 is produced.

Next, using an oscillation data recording device 5, the oscillation data recorded in the first mixed oscillation data recording water 4 is further discretely recorded in the oscillation data recording water 3 previously prepared in a predetermined number (10 units for example) of glass beakers 7 each being capable of storing a predetermined volume (10ml for example) of the oscillation data recording water 3, thereby reproducing the recording water 3d from the first mixed oscillation data recording water 4 by predetermined units (10 units for example).

Thenceforth, the recording water 3d reproduced from the first mixed oscillation data recording water 4 and stored in a predetermined number (10 units for example) of glass beakers 7a is then fully mixed into discretely prepared glass beakers 7b, thereby producing the second mixed oscillation data recording water 6.

Next, using the oscillation data recording device 5, oscillation data recorded in the second mixed oscillation data recording water 6 is further discretely recorded in the oscillation data recording water 3 previously stored in a predetermined number (10 units for example) of glass beakers 7 each having a predetermined volume (10ml for example), thereby further producing the recording water 3e reproduced from the second mixed oscillation data recording water 6 by predetermined units (10 units for example).

Thenceforth, by way of cyclically repeating the production processes comprising reproduction, mixing, and reproduction by predetermined rounds, the present invention has made it possible to produce novel oscillation data recording water internally recorded with such an oscillation data capable of supporting vitality of living bodies by enhancing the intensity of influence affecting living bodies.

Specifically, the novel method for generating oscillation data capable of supporting the vitality of individual living bodies proposed by the present invention adopts a form for preserving the oscillation data in the state being recorded in the oscillation data recording water by enabling individual oscillation data comprising the negative code, the positive code, and the intermediate code, respectively preset in the oscillation data measuring device (the MRA system), to be recorded based on an equivalent volume, followed by a mixing process after discretely dividing the data recording water into predetermined units of containers each having an equivalent volume capacity, thereby generating such an oscillation data mixed with three kinds of the oscillation data.

Hence, the inventive method provides such an advantage for enabling oscillation data generated from individual living bodies sustaining own living activities to be enhanced up to an intensely influential level by way of "directly strengthening self-sustaining of living bodies" while perfectly accommodating to the living bodies.

Further, the inventive method for generating oscillation data for supporting vitality of individual living bodies is compatible with the basic theory of the method for preparing remedy in the above-cited homeopathy medical treatment. It is acknowledged that, when applying the homeopathy medical treatment, by way of further repeating dilution with water, the influence of oscillation data on the living bodies becomes stronger. Hence, by way of cyclically repeating the production processes comprising reproduction, mixing, and reproduction of the oscillation data recording water by predetermined rounds, the present invention has made it possible to securely produce the novel oscillation data recording water internally recorded with such an oscillation data for supporting vitality of living bodies and being capable of enhancing the intensity of influence affecting living bodies.

### EFFECT OF THE INVENTION

As described above, in virtue of the novel method for generating oscillation data for supporting vitality of individual living bodies realized by the present invention,
the oscillation data recorded in the oscillation data recording water provides living bodies, by way of, for example, orally consuming the oscillation data recorded water, with such a distinguished effect of enhancing vitality of individual living bodies without causing them to incur unwanted burden as a foreign matter.

Further, the oscillation data recording water recorded with such an oscillation data capable of supporting vitality of living bodies can also be applied to a variety of industrial uses. For example, by way of applying the oscillation data recording water to the food processing industry, it is possible to provide the processed foods with a decreased burden against living bodies and an additional function for supporting vitality of the living bodies as well.

Further, by enabling the oscillation data to be recorded in the oscillation data recording water, the recorded data is free from incurring time-aged variation otherwise caused by the external environment. Hence, the inventive method has made it possible to simply and inexpensively produce novel oscillation data recording water by a large volume on the industrial basis, while providing the industrial fields with a variety of valuable effects via practical applicability thereof

## Claims

1. A method for generating oscillation data mainly aiming at supporting vitality of living bodies by preserving said data via recording in oscillation data recording water by applying an oscillation data recording device; said method comprising serial steps of;
producing three kinds of oscillation data recording water (3a), (3b), and (3c), by recording them in a predetermined volume of oscillation data recording water (3) by means of an oscillation data in-printer (2) pertaining to an oscillation data measuring device (1) previously storing an oscillation data containing negative properties, another oscillation data containing positive properties, and the other oscillation data containing intermediate properties therein;
further producing the first mixed oscillation data recording water (4) by mixing said three kinds of oscillation data recording water (3a, 3b, and 3c) with each other;
further producing predetermined plural units of the first mixed oscillation data recording water (3d) by causing oscillation data of said first mixed oscillation data recording water (4) to be recorded in said oscillation data recording water (3) comprising a predetermined volume and predetermined plural units;
further producing the second mixed oscillation data recording water (6) by fully mixing predetermined plural units of said first mixed oscillation data recording water (3d) with each other; and
completing production of said oscillation data recording water by cyclically repeating said reproducing, mixing, and reproducing processes by predetermined rounds.
